(19) Europäisches Patentamt
European Patent Office
Office européen des brevets

(11) Veröffentlichungsnummer: **0 629 401 A1**

# (12) EUROPÄISCHE PATENTANMELDUNG

(21) Anmeldenummer: **94108616.7**

(22) Anmeldetag: **06.06.94**

(51) Int. Cl.$^5$: **A61K 31/41**, C07D 285/00, C07D 417/12

(30) Priorität: **18.06.93 DE 4320157**

(43) Veröffentlichungstag der Anmeldung: **21.12.94 Patentblatt 94/51**

(84) Benannte Vertragsstaaten: **AT BE CH DE DK ES FR GB GR IE IT LI LU MC NL PT SE**

(71) Anmelder: **BAYER AG**
**D-51368 Leverkusen (DE)**

(72) Erfinder: **Heinemann, Ulrich, Dr.**
**Am Sonnenhang 1**
**D-42799 Leichlingen (DE)**
Erfinder: **Tiemann, Ralf, Dr.**
**Ernst-Ludwig-Kirchner-Strasse 5**
**D-51375 Leverkusen (DE)**
Erfinder: **Stünkel, Klaus, Dr.**
**Am Eckbusch 55**
**D-42113 Wuppertal (DE)**

## (54) Verwendung von 1,2,4-Dithia-zolium-Salzen als Chemotherapeutica insbesondere als TNF-Inhibitoren sowie neue 1,2,4-Dithiazolium-Salze.

(57) Die Erfindung betrifft die Verwendung von teilweise bekannten 1,2,4-Dithiazolium-Salzen der allgemeinen Formel (I)

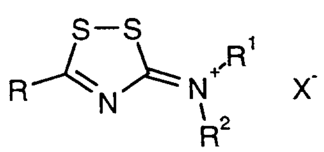

(I)

in welcher die Substituenten die in der Beschreibung angegebene Bedeutung haben,
ein Verfahren zu ihrer Herstellung und ihre Verwendung als Chemotherapeutica, insbesondere als TNF-Inhibitoren.

EP 0 629 401 A1

Die Erfindung betrifft die Verwendung von teilweise bekannten 1,2,4-Dithiazolium-Salzen, ein Verfahren zu ihrer Herstellung und ihre Verwendung als Chemotherapeutica.

Es wurde gefunden, daß die teilweise bekannten 1,2,4-Dithiazolium-Salze der allgemeinen Formel (I),

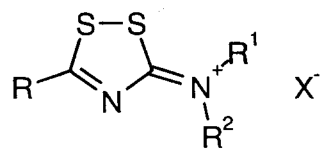

(I)

in welcher

| | |
|---|---|
| R | für einen Rest der Formel $-S-R^3$ oder $-NR^4R^5$ steht, |
| $R^1$ | für Alkyl oder Aryl steht und |
| $R^2$ | für Alkyl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann und |
| X | für das Anion einer anorganischen Säure steht, wobei |
| $R^3$ | für gegebenenfalls substituiertes Alkyl steht, |
| $R^4$ | für jeweils gegebenenfalls substituiertes Alkyl, Alkenyl, Alkinyl oder Aryl steht und |
| $R^5$ | für jeweils gegebenenfalls substituiertes Alkyl oder Alkenyl steht oder |
| $R^4$ und $R^5$ | gemeinsam mit dem Stickstoffatom, an welches sie gebunden sind, für einen gegebenenfalls substituierten Heterocyclus stehen, der gegebenenfalls weitere Heteroatome enthalten kann, |

eine gute Wirksamkeit als Chemotherapeutica besitzen und sich insbesondere als TNF-Inhibitoren verwenden lassen.

Tumor-Necrosis-Faktor-$\alpha$ (TNF-$\alpha$; Cachectin) und -$\beta$ (TNF-$\beta$; Lymphotoxin) sind verwandte Proteine, die von aktivierten Makrophagen bzw. aktivierten Lymphozyten gebildet werden (B. Beutler and A. Cerami, 1989, Annu. Rev. Immunol. 7: 625; C. Grunfeld and M.A. Palladine, 1990, Adv. Intern. Med. 35: 45). Wie in der Vergangenheit gezeigt werden konnte, sind diese Zytokine Bestandteil verschiedener biologischer Prozesse wie der Immunregulation, der Entzündung, der Cachexie, der Angiogenese und des septischen Schocks.

Die biologischen Effekte von TNF-$\alpha$ und TNF-$\beta$ werden durch spezifische Rezeptoren vermittelt. Klonierungsexperimente auf der Grundlage molekularbiologischer Methoden haben die Existenz zweier verschiedener TNF-Rezeptortypen (TNF-R) der molekularen Größe 55kDa und 75kDa belegen können und zwar für beide Spezies, Mensch und Maus. Dabei sind beide Rezeptortypen in der Lage sowohl TNF-$\alpha$ als auch TNF-$\beta$ als Ligand zu binden. Vertiefte Untersuchungen zeigten allerdings, daß humanes TNF-$\alpha$ ausschließlich Ligand für den murinen TNF-R des Molekulargewichtes 55kDa ist. Eine Bindung mit dem 75kDa TNF-R-Typ der Maus geht das humane TNF demzufolge nicht ein.

Verschiedene wissenschaftliche Mitteilungen der letzten Jahre versuchten die individuelle Rolle der beiden TNF-Rezeptoren zu beschreiben. Die Ergebnisse der Untersuchungen gehen überwiegend auf die Verwendung polyklonaler und monoklonaler Antikörper zurück, die gegen die lösliche Form beider TNF-R-Typen gerichtet waren. Gemäß dieser Untersuchungen lassen sich die folgenden TNF-vermittelten biologischen Funktionen mit dem 55kDa-TNF-R in Verbindung bringen: Zytotoxizität, Fibroblasten-Proliferation und Prostaglandin-$E_2$-Synthese. Die Rolle des 75kDa-TNF-R scheint nach ersten Berichten auf die Wachstumsstimulierung von Thymozyten und T-Lymphozyten (hier evtl. auch nur Subgruppen von T-Lymphozyten) sowie auch auf die von B-Lymphozyten beschränkt zu sein.

Die Pathogenese des endotoxemischen bzw. septischen Schocks scheint prädominant TNF-abhängig zu sein. Diese Aussage basiert auf verschiedenen experimentellen Beobachtungen:

1. Neutralisierende anti-TNF-$\alpha$-Antikörper verhindern Lungenversagen und Tod bei Mäusen und Affen (Baboons) als Resultat einer Endotoxin- oder E. coli-Applikation.
2. Die intravenöse Infusion von TNF-$\alpha$ führt zu einem toxikologischen Reaktionsmuster, das sich nicht von dem einer Endotoxemie bzw. einer Gram-negativen Sepsis unterscheidet.

Zusätzliche Hinweise für die besondere Rolle von TNF für das Schockgeschehen ergeben sich aus Befunden, die zeigen, daß bei Tieren und Menschen, die entweder Endotoxin oder einen septischen Schock bekommen hatten, die TNF-Serum-Spiegel signifikant erhöht waren im Vergleich zu unbelasteten Individuen. Im Fall von schwerer Sepsis korrelierten die erhöhten TNF-Serumspiegel mit dem Auftreten von

Mortalität.

Darüber hinaus verdichten sich Ergebnismitteilungen über die Bedeutung von Interleukin-1(IL-1)-neutralisierenden Prinzipien (z.B. IL-1-Rezeptorantagonist, IL-1 ra) für die Therapie des endotoxemischen bzw. septischen Schocks sowie anderer Indikationen.

In der Behandlung bakterieller Infektionen konnten in den letzten Jahren dank der Entwicklung potenter antibakterieller Substanzen bedeutende Fortschritte erzielt werden. Dessen ungeachtet erscheint die Anzahl an Sepsis- bzw. Mortalitätsfällen der letzten Jahre nach wie vor unerwartet hoch. Die Konsequenz aus solchen Beobachtungen ist der Versuch, neue Prinzipie/Verfahren zu entwickeln, die von einer antibakteriellen Chemotherapie abweichen. So konnten Teilerfolge mit einem humanen monoklonalen Antikörper gegen Endotoxin bei Patienten mit septischem Schock erreicht werden. Einen anderen therapeutischen Angang für septischen Schock stellt die Behandlung mit Hilfe eines murinen monoklonalen anti-humanen TNF-α-Antikörpers dar.

Neben diesen Aktivitäten auf dem Gebiet der Biologika hinsichtlich TNF-α- und IL-1-Antagonisten bzw. -Blockern gibt es Berichte zur Blockierung der TNF-Synthese oder TNF-α-Rezeptorbindung auf der Basis kleinmolekularer Substanzen (Pentoxifylline: U. Schade, 1990, Circ. Shock 31: 171; Tetrandrine: A. Ferrante et al., 1990, Clin. Exp. Immunol. 80: 232; Thalidomide: E.P. Sampaio et al., 1991, J. Exp. Med. 173: 699; Colchicine: G. Tiegs et al., 1992, Infect. Immun. 60: 1941).

Die erfindungsgemäß verwendbaren 1,2,4-Dithiazolium-Salze sind durch die Formel (I) allgemein definiert. Bevorzugt verwendbar sind Verbindungen der Formel (I), bei welchen

R für einen Rest der Formel -S-$R^3$ oder -$NR^4R^5$ steht,

$R^1$ für geradkettiges oder verzweigtes Alkyl mit 1 bis 8 Kohlenstoffatomen oder für gegebenenfalls einfach oder mehrfach, gleich oder verschieden substituiertes Aryl mit 6 bis 10 Kohlenstoffatomen steht, wobei als Substituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl und

$R^2$ für geradkettiges oder verzweigtes Alkyl mit 1 bis 8 Kohlenstoffatomen steht oder

$R^1$ und $R^2$ gemeinsam mit dem Stickstoffatom, an welches sie gebunden sind für einen gegebenenfalls einfach oder mehrfach, gleich oder verschieden substituierten, gesättigten fünf- bis siebengliedrigen Heterocyclus stehen, der gegebenenfalls weitere Heteroatome - insbesondere Stickstoff, Sauerstoff und/oder Schwefel - enthalten kann, wobei als Substituenten infrage kommen:
Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen,

X für das Anion einer anorganischen Säure steht, wobei

$R^3$ für geradkettiges oder verzweigtes Alkyl mit 1 bis 8 Kohlenstoffatomen oder für jeweils gegebenenfalls im Arylteil einfach oder mehrfach, gleich oder verschieden substituiertes Arylalkyl mit 6 bis 10 Kohlenstoffatomen im Arylteil und 1 bis 4 Kohlenstoffatomen im geradkettigen oder verzweigten Alkylteil oder Heteroarylalkyl mit 2 bis 9 Kohlenstoffatomen und 1 bis 3 gleichen oder verschiedenen Heteroatomen - insbesondere Stickstoff, Sauerstoff und/oder Schwefel - im Heteroarylteil und 1 bis 4 Kohlenstoffatomen im geradkettigen oder verzweigten Alkylteil steht, wobei als Substituenten jeweils infrage kommen:
Halogen, Cyano, Nitro, Carbamoyl, jeweils geradkettiges oder verzweigtes Alkyl, Alkoxy oder Alkoxycarbonyl mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen,

$R^4$ für jeweils geradkettiges oder verzweigtes Alkyl, Alkenyl oder Alkinyl mit jeweils bis zu 8

Kohlenstoffatomen, für geradkettiges oder verzweigtes Halogenalkyl mit 1 bis 8 Kohlenstoffatomen und 1 bis 17 gleichen oder verschiedenen Halogenatomen, für jeweils geradkettiges oder verzweigtes Cyanalkyl, Dioxolanylalkyl, Alkoxyalkyl oder Dialkylaminoalkyl mit jeweils 1 bis 8 Kohlenstoffatomen in den einzelnen Alkylteilen, für Cycloalkylalkyl mit 3 bis 8 Kohlenstoffatomen im Cycloalkylteil und 1 bis 8 Kohlenstoffatomen im geradkettigen oder verzweigten Alkylteil oder für jeweils gegebenenfalls im Arylteil einfach oder mehrfach, gleich oder verschieden substituiertes Aryl mit 6 bis 10 Kohlenstoffatomen, Arylalkyl mit 6 bis 10 Kohlenstoffatomen im Arylteil und 1 bis 4 Kohlenstoffatomen im geradkettigen oder verzweigten Alkylteil oder Heteroarylalkyl mit 2 bis 9 Kohlenstoffatomen und 1 bis 3 gleichen oder verschiedenen Heteroatomen - insbesondere Stickstoff, Sauerstoff und/oder Schwefel - im Heteroarylteil und 1 bis 4 Kohlenstoffatomen im geradkettigen oder verzweigten Alkylteil steht, wobei als Substituenten jeweils infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen und

$R^5$ für jeweils geradkettiges oder verzweigtes Alkyl oder Alkenyl mit jeweils bis zu 8 Kohlenstoffatomen steht oder für gegebenenfalls im Arylteil einfach oder mehrfach, gleich oder verschieden substituiertes Arylalkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder

$R^4$ und $R^5$ gemeinsam mit dem Stickstoffatom, an welches sie gebunden sind für einen gegebenenfalls einfach oder mehrfach, gleich oder verschieden substituierten, gesättigten fünf- bis siebengliedrigen Heterocyclus stehen, der gegebenenfalls weitere Heteroatome - insbesondere Stickstoff, Sauerstoff und/oder Schwefel - enthalten kann, wobei als Substituenten infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen.

Besonders bevorzugt verwendbar sind Verbindungen der Formel (I), bei welchen

R für einen Rest der Formel $-S-R^3$ oder $-NR^4R^5$ steht,

$R^1$ für geradkettiges oder verzweigtes Alkyl mit 1 bis 6 Kohlenstoffatomen oder für gegebenenfalls einfach bis dreifach, gleich oder verschieden substituiertes Phenyl steht, wobei als Substituenten jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen,

$R^2$ für geradkettiges oder verzweigtes Alkyl mit 1 bis 6 Kohlenstoffatomen steht oder

$R^1$ und $R^2$ gemeinsam mit dem Stickstoffatom, an welches sie gebunden sind für einen gegebenenfalls einfach bis vierfach, gleich oder verschieden substituierten, gesättigten fünf- bis siebengliedrigen Heterocyclus stehen, der gegebenenfalls ein weiteres Heteroatom - insbesondere Stickstoff, Sauerstoff oder Schwefel - enthalten kann, wobei als Substituenten infrage kommen:

Halogen oder jeweils geradkettiges oder verzweigtes Alkyl oder Alkoxy mit jeweils 1 bis 4 Kohlenstoffatomen,

X für ein Halogenidanion, ein Phosphatanion, ein Hexafluorophosphatanion, ein Sulfatanion, ein Hydrogensulfatanion, ein Nitratanion, ein Carbonatanion, ein Perchloratanion oder ein Tetrafluoroboratanion steht, wobei

$R^3$ für geradkettiges oder verzweigtes Alkyl mit 1 bis 4 Kohlenstoffatomen oder für jeweils gegebenenfalls im Arylteil einfach bis dreifach, gleich oder verschieden substituiertes

Arylalkyl mit 6 oder 10 Kohlenstoffatomen im Arylteil und 1 oder 2 Kohlenstoffatomen im Alkylteil oder Heteroarylalkyl mit 2 bis 5 Kohlenstoffatomen und 1 bis 3 gleichen oder verschiedenen Heteroatomen - insbesondere Stickstoff, Sauerstoff und/oder Schwefel - im Heteroarylteil und 1 oder 2 Kohlenstoffatomen im Alkylteil steht, wobei als Substituenten jeweils infrage kommen:

Fluor, Chlor, Brom, Iod, Cyano, Nitro, Carbamoyl, jeweils geradkettiges oder verzweigtes Alkyl, Alkoxy oder Alkoxycarbonyl mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen,

$R^4$ für jeweils geradkettiges oder verzweigtes Alkyl, Alkenyl oder Alkinyl mit jeweils bis zu 6 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Cyanalkyl, Dioxolanylalkyl, Alkoxyalkyl oder Dialkylaminoalkyl mit jeweils 1 bis 4 Kohlenstoffatomen in den einzelnen Alkylteilen, für Cycloalkylalkyl mit 3 bis 7 Kohlenstoffatomen im Cycloalkylteil und 1 bis 4 Kohlenstoffatomen im geradkettigen oder verzweigten Alkylteil oder für jeweils gegebenenfalls im Arylteil einfach bis dreifach, gleich oder verschieden substituiertes Aryl mit 6 oder 10 Kohlenstoffatomen, Arylalkyl mit 6 oder 10 Kohlenstoffatomen im Arylteil und 1 oder 2 Kohlenstoffatomen im Alkylteil oder Heteroarylalkyl mit 2 bis 5 Kohlenstoffatomen und 1 bis 3 gleichen oder verschiedenen Heteroatomen - insbesondere Stickstoff, Sauerstoff und/oder Schwefel - im Heteroarylteil und 1 oder 2 Kohlenstoffatomen im Alkylteil steht, wobei als Substituenten jeweils infrage kommen:

Fluor, Chlor, Brom, Iod, jeweils geradkettiges oder verzweigtes Alkyl oder Alkoxy mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen und

$R^5$ für jeweils geradkettiges oder verzweigtes Alkyl oder Alkenyl mit jeweils bis zu 6 Kohlenstoffatomen steht oder für gegebenenfalls im Arylteil einfach bis dreifach, gleich oder verschieden substituiertes Arylalkyl mit 6 oder 10 Kohlenstoffatomen im Arylteil und 1 oder 2 Kohlenstoffatomen im Alkylteil steht, wobei als Substituenten infrage kommen:

Fluor, Chlor, Brom, Iod, jeweils geradkettiges oder verzweigtes Alkyl oder Alkoxy mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen oder

$R^4$ und $R^5$ gemeinsam mit dem Stickstoffatom, an welches sie gebunden sind für einen gegebenenfalls einfach bis vierfach, gleich oder verschieden substituierten, gesättigten fünf- bis siebengliedrigen Heterocyclus stehen, der gegebenenfalls ein weiteres Heteroatom - insbesondere Stickstoff, Sauerstoff oder Schwefel - enthalten kann, wobei als Substituenten infrage kommen:

Halogen oder jeweils geradkettiges oder verzweigtes Alkyl oder Alkoxy mit jeweils 1 bis 4 Kohlenstoffatomen.

Ganz besonders bevorzugt verwendbar sind Verbindungen der Formel (I), bei welchen

R für einen Rest der Formel $-S-R^3$ oder $-NR^4R^5$ steht,

$R^1$ für geradkettiges oder verzweigtes Alkyl mit 1 bis 4 Kohlenstoffatomen oder für gegebenenfalls einfach oder zweifach, gleich oder verschieden substituiertes Phenyl steht, wobei als Substituenten jeweils infrage kommen:

Fluor, Chlor, Brom, Cyano, Nitro, Methyl, Ethyl, n-oder i-Propyl, n-, i-, s-oder t-Butyl, Methoxy, Ethoxy, n-oder i-Propoxy, n-, i-, s-oder t-Butoxy, Trifluormethyl, Difluormethyl, Trifluormethoxy oder Difluormethoxy,

$R^2$ für geradkettiges oder verzweigtes Alkyl mit 1 bis 4 Kohlenstoffatomen steht oder

$R^1$ und $R^2$ gemeinsam mit dem Stickstoffatom, an welches sie gebunden sind für einen gegebenenfalls einfach oder zweifach substituierten Pyrrolidinyl-, Piperidinyl-, Perhydroazepinyl-, Piperazinyl- oder Morpholinylrest stehen, wobei als Substituenten jeweils infrage kommen:

Chlor, Methyl, Ethyl, Methoxy oder Ethoxy,

X für ein Fluorid-, Chlorid-, Bromid-, Iodid- oder Tetrafluoroboratanion steht, wobei

$R^3$ für Methyl, Ethyl oder für jeweils gegebenenfalls im Arylteil einfach oder zweifach, gleich oder verschieden substituiertes Phenyl, Naphthyl, Benzyl oder Pyridylmethyl steht, wobei als Substituenten jeweils infrage kommen:

Fluor, Chlor, Brom, Cyano, Nitro, Methyl, Ethyl, n-oder i-Propyl, Methoxy, Ethoxy, n-oder i-Propoxy, Methoxycarbonyl, Ethoxycarbonyl, Carbamoyl, Trifluormethyl, Difluormethyl, Trifluormethoxy oder Difluormethoxy,

$R^4$ für Methyl, Ethyl, n- oder i-Propyl, n-, i-, s- oder t-Butyl, Allyl, Propargyl, Cyanethyl, Methoxyethyl, Ethoxyethyl, Dimethylaminoethyl, Diethylaminoethyl, Dioxolanylmethyl, Cyclopropylmethyl, Cyclohexylmethyl oder für jeweils gegebenenfalls im Arylteil einfach oder zweifach, gleich oder verschieden substituiertes Phenyl, Naphthyl, Benzyl, Phenylethyl oder Pyridylmethyl steht, wobei als Substituenten jeweils infrage kommen:

Fluor, Chlor, Brom, Methyl, Ethyl, n-oder i-Propyl, Methoxy, Ethoxy, n-oder i-Propoxy, Trifluormethyl, Difluormethyl, Trifluormethoxy oder Difluormethoxy und

$R^5$ für Methyl, Ethyl, n- oder i-Propyl, n-, i-, s- oder t-Butyl, Allyl oder für gegebenenfalls im Arylteil einfach oder zweifach, gleich oder verschieden substituiertes Benzyl steht, wobei als Substituenten infrage kommen: Fluor, Chlor, Brom, Methyl, Ethyl, n-oder i-Propyl, Methoxy, Ethoxy, n-oder i-Propoxy, Trifluormethyl, Difluormethyl, Trifluormethoxy oder Difluormethoxy oder

$R^4$ und $R^5$ gemeinsam mit dem Stickstoffatom, an welches sie gebunden sind für einen gegebenenfalls einfach oder zweifach substituierten Pyrrolidinyl-, Piperidinyl-, Perhydroazepinyl-, Piperazinyl- oder Morpholinylrest stehen, wobei als Substituenten jeweils infrage kommen: Chlor, Methyl, Ethyl, Methoxy oder Ethoxy.

Im einzelnen seien außer den bei den Herstellungsbeispielen genannten Verbindungen die folgenden 1,2,4-Dithiazolium-Salze der allgemeinen Formel (I) genannt:

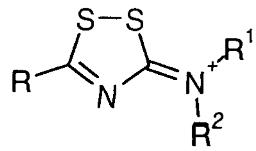 $X^-$ (I)

| R | $R^1$ | $R^2$ | X |
|---|---|---|---|
| $C_2H_5$-S- | $CH_3$ | $CH_3$ | $I^-$ |
| n-$C_3H_7$-S- | $CH_3$ | $CH_3$ | $I^-$ |
| 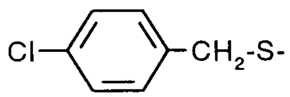 | $CH_3$ | $CH_3$ | $Br^-$ |
| 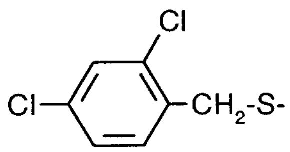 | $CH_3$ | $CH_3$ | $Br^-$ |
| 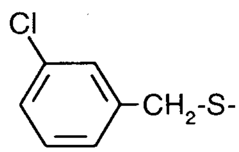 | $CH_3$ | $CH_3$ | $BF_4^-$ |
| 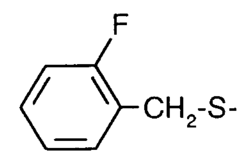 | $CH_3$ | $CH_3$ | $BF_4^-$ |
| 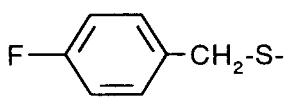 | $CH_3$ | $CH_3$ | $BF_4^-$ |
| 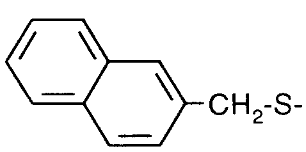 | $CH_3$ | $CH_3$ | $Br^-$ |

| R | $R^1$ | $R^2$ | X |
|---|---|---|---|
| 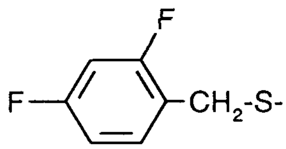 | $-CH_2-(CH_2)_3-CH_2-$ | | $Br^-$ |
| 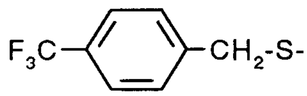 | $-CH_2-(CH_2)_3-CH_2-$ | | $BF_4^-$ |
| 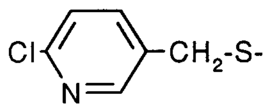 | $-CH_2-(CH_2)_3-CH_2-$ | | $BF_4^-$ |
| $CH_3$-S- | $-CH_2-(CH_2)_3-CH_2-$ | | $I^-$ |
| $CH_3$-S- | $C_2H_5$ | $C_2H_5$ | $I^-$ |
| $C_2H_5$-S- | $C_2H_5$ | $C_2H_5$ | $I^-$ |
| $C_6H_5-CH_2$-S- | $C_2H_5$ | $C_2H_5$ | $BF_4^-$ |
| $(C_2H_5)_2N$- | $-CH_2-(CH_2)_3-CH_2-$ | | $I^-$ |
| $(n-C_3H_7)_2N$- | $-CH_2-(CH_2)_3-CH_2-$ | | $I^-$ |
| $(i-C_3H_7)_2N$- | $-CH_2-(CH_2)_3-CH_2-$ | | $I^-$ |
| 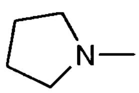 | $-CH_2-(CH_2)_3-CH_2-$ | | $I^-$ |
| 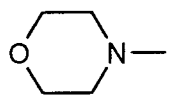 | $-CH_2-(CH_2)_3-CH_2-$ | | $I^-$ |
| 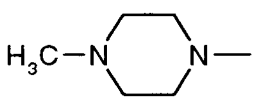 | $-CH_2-(CH_2)_3-CH_2-$ | | $I^-$ |
| 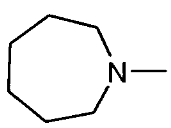 | $-CH_2-(CH_2)_3-CH_2-$ | | $I^-$ |

| R | $R^1$ | $R^2$ | X |
|---|---|---|---|
| 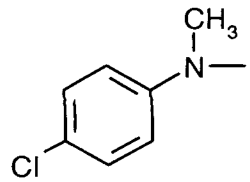 | $-CH_2-(CH_2)_3-CH_2-$ | | $BF_4^-$ |
| 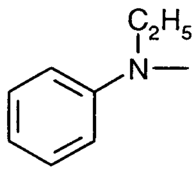 | $-CH_2-(CH_2)_3-CH_2-$ | | $BF_4^-$ |
| 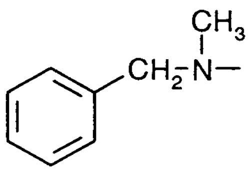 | $-CH_2-(CH_2)_3-CH_2-$ | | $I^-$ |
| 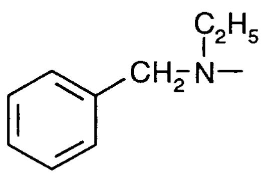 | $-CH_2-(CH_2)_3-CH_2-$ | | $I^-$ |
| 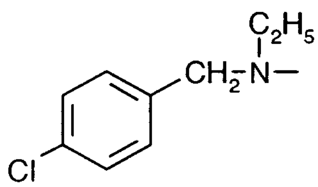 | $-CH_2-(CH_2)_3-CH_2-$ | | $I^-$ |
| $(CH_2=CH-CH_2)_2N-$ | $-CH_2-(CH_2)_3-CH_2-$ | | $BF_4^-$ |
| $(n-C_4H_9)_2N-$ | $-CH_2-(CH_2)_3-CH_2-$ | | $I^-$ |
| $(i-C_4H_9)_2N-$ | $-CH_2-(CH_2)_3-CH_2-$ | | $I^-$ |
| 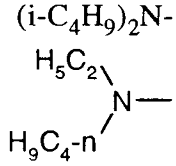 | $-CH_2-(CH_2)_3-CH_2-$ | | $BF_4^-$ |
| 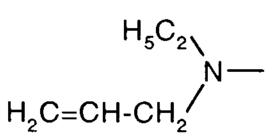 | $-CH_2-(CH_2)_3-CH_2-$ | | $I^-$ |

| R | $R^1$ | $R^2$ | X |
|---|---|---|---|
| 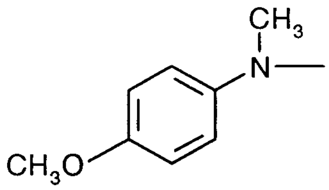 | $-CH_2-(CH_2)_2-CH_2-$ | | $I^-$ |
| 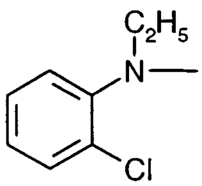 | $-CH_2-(CH_2)_2-CH_2-$ | | $BF_4^-$ |
| 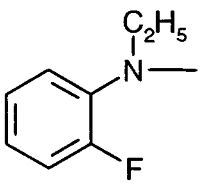 | $-CH_2-(CH_2)_2-CH_2-$ | | $BF_4^-$ |
| 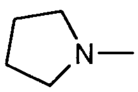 | $C_2H_5$ | $C_2H_5$ | $I^-$ |
| 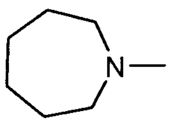 | $C_2H_5$ | $C_2H_5$ | $BF_4^-$ |
| 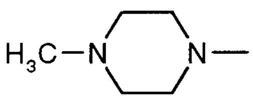 | $C_2H_5$ | $C_2H_5$ | $I^-$ |
| 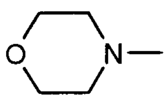 | $C_2H_5$ | $C_2H_5$ | $I^-$ |
| 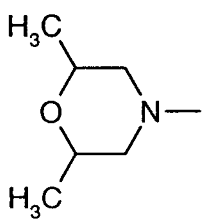 | $C_2H_5$ | $C_2H_5$ | $I^-$ |

| R | $R^1$ | $R^2$ | X |
|---|---|---|---|
| 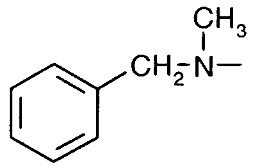 | $C_2H_5$ | $C_2H_5$ | $I^-$ |
| 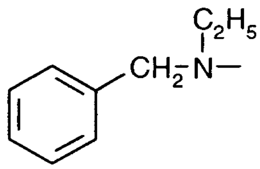 | $C_2H_5$ | $C_2H_5$ | $I^-$ |
| $(C_6H_5\text{-}CH_2)_2N\text{-}$ | $C_2H_5$ | $C_2H_5$ | $I^-$ |
| 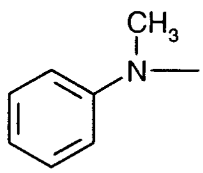 | $C_2H_5$ | $C_2H_5$ | $BF_4^-$ |
| 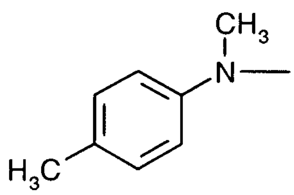 | $C_2H_5$ | $C_2H_5$ | $I^-$ |

Die erfindungsgemäß verwendbaren 1,2,4-Dithiazolium-Salze der Formel (I) sind teilweise bekannt (vergl. z.B. J. Org. Chem. 36, 3465 [1971]; J. Med. Chem. 15, 315-320 [1972]; J. Econ. Entomol. 65, 390-392 [1972]; Bull. Soc. Chim. Fr. 9-10 Pt.2, 481-484 [1978]; JP 55081804; JP 57183770; Bull. Soc. Chem. Japan 60, 2686-2688 [1987]; DE 42 27 751).

Man erhält sie, wenn man 1,2,4-Dithiazolin-5-thione der Formel (II),

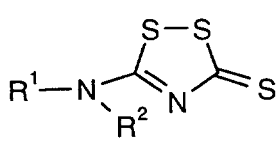 (II)

in welcher

$R^1$ und $R^2$ die oben angegebene Bedeutung haben,

mit Alkyliodiden der Formel (III),

$R^3$- I (III)

in welcher

$R^3$ die oben angegebene Bedeutung hat,

gegebenenfalls in Gegenwart eines Verdünnungsmittels umsetzt und gegebenenfalls anschließend die so erhältlichen 1,2,4-Dithiazolium-Salze der Formel (Ia),

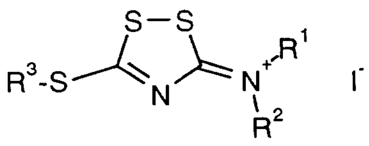

(Ia)

in welcher

$R^1$, $R^2$ und $R^3$ die oben angegebene Bedeutung haben,

in einer anschließenden zweiten Stufe mit Aminen der Formel (IV),

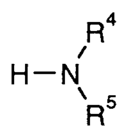

(IV)

in welcher

$R^4$ und $R^5$ die oben angegebene Bedeutung haben,

gegebenenfalls in Gegenwart eines Verdünnungsmittels umsetzt und gegebenenfalls anschließend durch Umsetzung mit einer anorganischen Säure oder einem entsprechenden Salz das Anion austauscht.

Verwendet man beispielsweise 3-Dimethylamino-1,2,4-dithiazolin-5-thion, Methyliodid und 2,6-Dimethylmorpholin als Ausgangsstoffe, so läßt sich der Reaktionsablauf des Herstellungsverfahrens durch das folgende Formelschema darstellen:

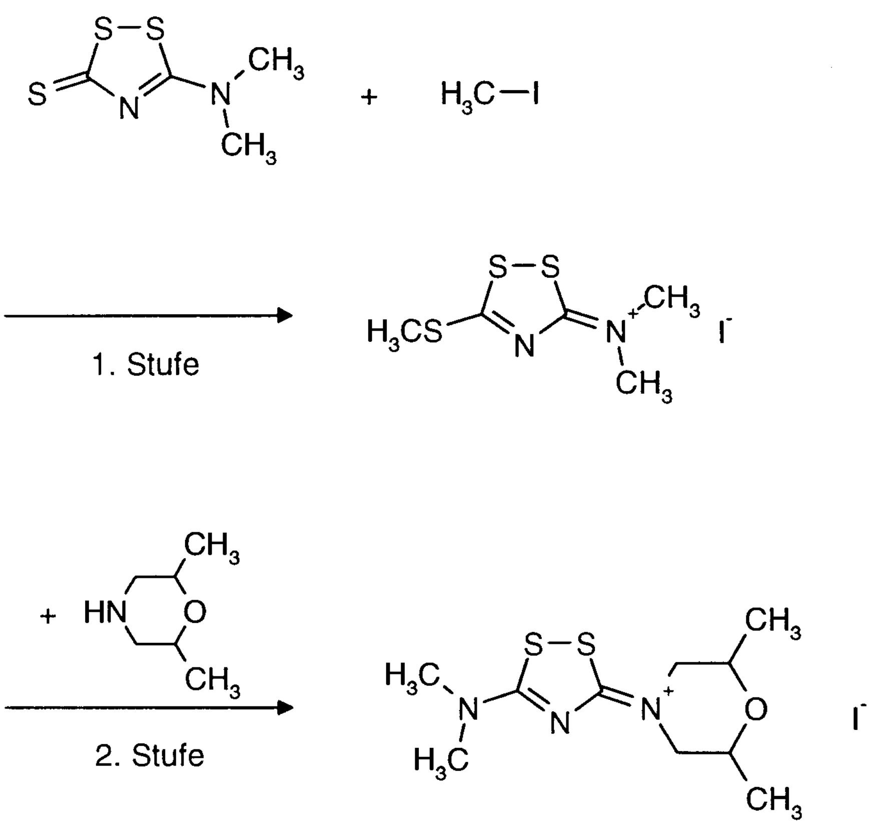


Die zur Durchführung des Herstellungsverfahrens als Ausgangsstoffe benötigten 1,2,4-Dithiazolin-5-thione sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäß verwendbaren Verbindungen der Formel (I) als bevorzugt für diese Substituenten genannt wurden.

1,2,4-Dithiazolin-5-thione der Formel (II) sind bekannt oder erhältlich in Analogie zu bekannten Verfahren (vergl. z.B. J. Amer. Chem. Soc. 80, 414 [1958]; Liebigs Ann. Chem. 285, 174 [1895]).

Die zur Durchführung des Herstellungsverfahrens weiterhin als Ausgangsstoffe benötigten Alkyliodide sind durch die Formel (III) allgemein definiert. In dieser Formel (III) steht $R^3$ vorzugsweise für diejenigen Reste, die bereits im Zusammenhang mit der Beschreibung der erfindungsgemäß verwendbaren Verbindungen der Formel (I) als bevorzugt für diesen Substituenten genannt wurden. Alkyliodide der Formel (III) sind allgemein bekannte Verbindungen der organischen Chemie.

Die zur Durchführung des Herstellungsverfahrens gegebenenfalls weiterhin als Ausgangsstoffe benötigten Amine sind durch die Formel (IV) allgemein definiert. In dieser Formel (IV) stehen $R^4$ und $R^5$ vorzugsweise für diejenigen Reste, die bereits im Zusammenhang mit der Beschreibung der erfindungsgemäß verwendbaren Verbindungen der Formel (I) als bevorzugt für diese Substituenten genannt wurden. Amine der Formel (IV) sind allgemein bekannte Verbindungen der organischen Chemie.

Noch nicht bekannt und ebenfalls Gegenstand der Erfindung sind 1,2,4-Dithiazolium-Salze der Formel (Ib),

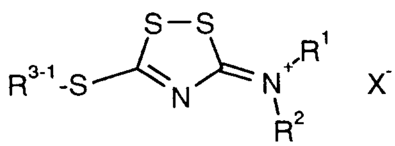

(Ib)

in welcher

| | |
|---|---|
| $R^1$ | für Alkyl oder Aryl steht und |
| $R^2$ | für Alkyl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann und |
| X | für das Anion einer anorganischen Säure steht, wobei |
| $R^{3-1}$ | für jeweils gegebenenfalls substituiertes Arylalkyl oder Heterocyclylalkyl steht. |

Als Verdünnungsmittel zur Durchführung der 1. und der 2. Stufe des Herstellungsverfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl-oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Sulfoxide, wie Dimethylsulfoxid, oder Sulfone wie Sulfolan.

Die Reaktionstemperaturen können bei der Durchführung der 1. Stufe des Herstellungsverfahrens in einem größeren Bereich variiert werden. Im allgemeinen arbeitet man bei Temperaturen zwischen 0 °C und 140 °C, vorzugsweise bei Temperaturen zwischen 20 °C und 120 °C.

Die Reaktionstemperaturen können bei der Durchführung der 2. Stufe des Herstellungsverfahrens in einem größeren Bereich variiert werden. Im allgemeinen arbeitet man bei Temperaturen zwischen 0 °C und 140 °C, vorzugsweise bei Temperaturen zwischen 20 °C und 80 °C.

Die 1. und die 2. Stufe des Herstellungsverfahrens werden üblicherweise unter Normaldruck durchgeführt. Es ist jedoch auch möglich unter erhöhtem oder vermindertem Druck zu arbeiten.

Zur Durchführung der 1. Stufe des Herstellungsverfahrens setzt man pro Mol an 1,2,4-Dithiazolin-5-thion der Formel (II) im allgemeinen 1,0 bis 1,5 Mol, vorzugsweise 1,0 bis 1,1 Mol an Alkyliodid der Formel (III) ein. Die Reaktionsdurchführung, Aufarbeitung und Isolierung der Reaktionsprodukte erfolgt nach üblichen Verfahren (vergl. hierzu auch die Herstellungsbeispiele).

Zur Durchführung der 2. Stufe des Herstellungsverfahrens setzt man pro Mol an 1,2,4-Dithiazolium-Iodid der Formel (Ia) im allgemeinen 1,0 bis 1,5 Mol, vorzugsweise 1,0 bis 1,1 Mol an Amin der Formel (IV) ein. Die Reaktionsdurchführung, Aufarbeitung und Isolierung der Reaktionsprodukte erfolgt nach üblichen Verfahren (vergl. hierzu auch die Herstellungsbeispiele).

Die Reinigung der Endprodukte der Formel (I) erfolgt ebenfalls mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Im folgenden werden exemplarisch Substanzen aus der Substanzklasse der Dithiazoliumsalze in ihrer protektiven Wirkung beschrieben: in-vitro im TNF-α-vermittelten Zytotoxizitätstest, in-vivo bei Mäusen, die einem Endotoxin-Schock ausgesetzt wurden.

Der TNF-α-vermittelte Zytotoxizitätstest wurde unter Verwendung der Zellinie WEHI-164-Klon-13, einer Fibrosarcoma-Linie, im wesentlichen nach der Arbeitsanleitung von Espevik und Nissen-Meyer 1986 durchgeführt (J. Immunol. Methods 95: 99-105). Hierzu wurden 2,5 x $10^4$ Zellen pro Vertiefung einer 96-Kavitätenzählenden Mikrotiterplatte unter Verwendung von RPMI 1640 und 5 % fötalem Kälberserum einer 5-stündigen Vorinkubation unterworfen. Nach Ablauf der Zeit wurden verschiedene Substanzmengen eines Dithiazoliumsalzes (3-10-30-100 µg/ml), Actinomycin (4 µg/ml) und TNF-α (25 µg/ml) den Zellen zugesetzt und einer weiteren Inkubation für 18 Std. unterzogen.

Vitalitätsbestimmungen erfolgten mit Hilfe eines colorimetrischen Verfahrens unter Verwendung von 3-[4,5-Dimethylthiazol-2-yl]-2,5-diphenyltetrazolium Bromide (MTT) in einem Mikrotiterplatten-Auswertsystem bei einer Wellenlänge von 570 nm und einer Referenzwellenlänge von 630 mm (in Anlehnung an Mosmann, 1983, J. Immunol. Methods 65: 55).

Versuche in-vivo zur Hemmung des provozierten Endotoxin-Schocks wurden an weiblichen Inzucht-Mäusen vorgenommen ($F_1$[B6D2]). Endotoxin/Lipopolysaccharide (LPS) von Salmonella abortus equi (0,1 µg/Maus) wurde in Verbindung mit D-Galaktosamin-hydrochlorid (D-GalN; 60 mg/kg Körpergewicht) intraperitoneal oder intravenös appliziert in Anlehnung an Mitteilungen von Galanos et al. (1979, Proc. Natl. Acad. Sci. USA 76: 5939; V. Lehmann et al. 1987, J. Exp. Med. 165: 657). Die Applikation der Substanzen erfolgte eine Stunde vor Auslösung des endotoxemischen Schocks intraperitoneal. Der Beobachtungszeitraum betrug 48 Stunden.

Tabelle 1: Einfluß von Dithiazoliumsalzen auf den Verlauf des endotoxemischen Schocks bei D-GalN-sensibilisierten Mäusen bzw. auf die TNF-α-vermittelte Zytotoxizität von Zellen der Zellinie WEHI 164-Klon 13.

| Substanz Beispiel | in-vivo Dosis (mg/kg) | | | | | in-vitro[1)] |
|---|---|---|---|---|---|---|
| | LPS + D-GaIN [Kontrolle] | Substanz[2)] + D-GaIN [Kontrolle] | 10 | 30 | 60 | |
| | | | Letalität | | | |
| 10 | 100 | 0 | 20 | 0 | 0 | + |
| 5 | 100 | 0 | 40 | 0 | 0 | + |
| 3 | 100 | 0 | 80 | 40 | 0 | +/- |
| 8 | 100 | 0 | 40 | 0 | 0 | +/- |
| 2 | 100 | 0 | 100 | 20 | 0 | + |
| 43 | 100 | 0 | 20 | 20 | 0 | n.d.[3)] |
| 46 | 100 | 0 | 0 | 0 | 0 | n.d. |
| 48 | 100 | 0 | 0 | 0 | 0 | n.d. |

[1)] Hemmung der TNF-α-vermittelten Lyse der Zielzellen

[2)] 60 mg/kg

[3)] nicht durchgeführt

Die protektiven Eigenschaften können darüber hinaus beispielsweise auf einer TNF-α/β oder/und IL-1α/β-Synthesehemmung, auf einer TNF-α/β- oder/und IL-1 α/β-Rezeptorblockade, auf einer Hemmung der Leukozytenadhärenz an das Endothelium oder auch auf anderen Mechanismen beruhen.

**Herstellungsbeispiele:**

Beispiel 1:

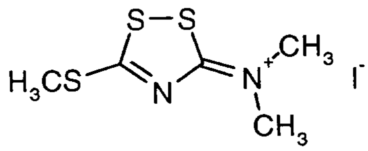

44,5 g (0,252 Mol) 3-Dimethylamino-1,2,4-dithiazolin-5-thion und 39,1 g (0,275 Mol) Methyliodid in 350 ml Acetonitril werden für 5 Stunden auf Rückflußtemperatur erhitzt. Zur Aufarbeitung wird die Reaktionsmischung abgekühlt, und die ausgefallenen Kristalle werden abgesaugt und getrocknet.

Man erhält 71,8 g (90 % der Theorie) an 3-Dimethylamino-5-methylthio-1,2,4-dithiazolium Iodid vom Schmelzpunkt 161 ° C (Zers.).

Beispiel 2:

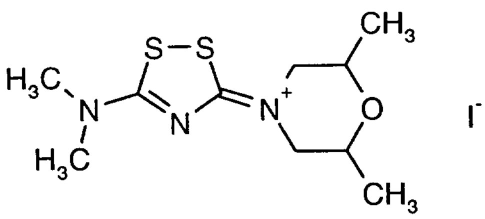

6,4 g (0,02 Mol) 3-Dimethylamino-5-methylthio-1,2,4-dithiazolium-iodid und 2,3 g (0,02 Mol) 2,6-Dimethylmorpholin in 50 ml Dichlormethan werden für 16 Stunden auf Rückflußtemperatur erhitzt. Zur Aufarbeitung wird die abgekühlte Reaktionsmischung im Vakuum eingeengt, der Rückstand mit heißem Essigester verrührt und abgekühlt. Die ausgefallenen Kristalle werden abgesaugt und getrocknet.

Man erhält 6,7 g g (87 % der Theorie) an 4-(5-Dimethylamino-3H-1,2,4-dithiazol-3-yliden)-2,6-dimethylmorpholinium Iodid vom Schmelzpunkt 192 ° C (Zers.).

Beispiel 3:

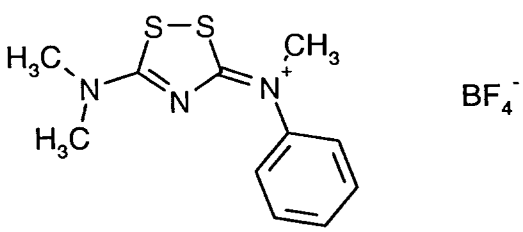

6,4 g (0,02 Mol) 3-Dimethylamino-5-methylthio-1,2,4-dithiazolium-iodid und 2,1 g (0,02 Mol) N-Methylanilin in 50 ml Chloroform werden für 36 Stunden auf Rückflußtemperatur erhitzt. Zur Aufarbeitung wird die abgekühlte Reaktionsmischung im Vakuum eingeengt, der Rückstand in Dichlormethan aufgenommen, mit einer wässrigen Lösung von Natriumtetrafluoroborat gerührt, die organische Phase abgetrennt, zweimal mit Wasser gewaschen, über Natriumsulfat getrocknet und im Vakuum eingeengt.

Man erhält 5,5 g (81 % der Theorie) an N-(5-Dimethylamino-3H-1,2,4-dithiazol-3-yliden)-N-methylanilinium Tetrafluoroborat vom Schmelzpunkt 128 ° C (Zers.).

In entsprechender Weise und gemäß den allgemeinen Angaben zur Herstellung erhält man die folgenden 1,2,4-Dithiazolium-Salze der allgemeinen Formel (I):

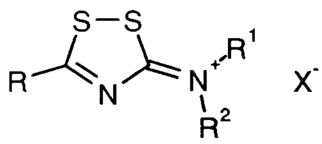

(I)

| Bsp.-Nr. | R | $R^1$ | $R^2$ | X | physikalische Eigenschaften |
|---|---|---|---|---|---|
| 4 | $-N(CH_3)_2$ | $-CH_2-(CH_2)_3-CH_2-$ | | $I^-$ | Fp. 87°C (Zers.) |
| 5 | $-N(CH_3)_2$ | $-(CH_2)_2-O-(CH_2)_2-$ | | $I^-$ | $^1$H-NMR*): 3,3; 3,45 (d, 6H) |
| 6 | $-N(CH_3)_2$ | $n-C_3H_7$ | $n-C_3H_7$ | $I^-$ | Fp. 111-113°C (Zers.) |
| 7 | $-N(CH_3)_2$ | $-CH_2-(CH_2)_2-CH_2-$ | | $I^-$ | Fp. 89°C (Zers.) |
| 8 | $-N(CH_3)_2$ | $-(CH_2)_2-N(CH_3)-(CH_2)_2-$ | | $I^-$ | Fp. 169°C (Zers.) |
| 9 | $-N(CH_3)_2$ | $C_2H_5$ | $C_2H_5$ | $I^-$ | Fp. 127°C (Zers.) |
| 10 | $-N(CH_3)_2$ | $i-C_3H_7$ | $i-C_3H_7$ | $I^-$ | Fp. 132°C (Zers.) |
| 11 | $-N(CH_3)_2$ | $n-C_4H_9$ | $n-C_4H_9$ | $I^-$ | $^1$H-NMR*): 3,25; 3,4 (d, 6H) |
| 12 | $-N(CH_3)_2$ | $i-C_4H_9$ | $i-C_4H_9$ | $I^-$ | Fp. 99-102°C (Zers.) |
| 13 | 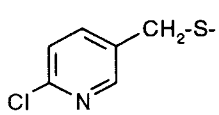 | $CH_3$ | $CH_3$ | $BF_4^-$ | $^1$H-NMR*): 3,25; 3,35 (d, 6H) |
| 14 | 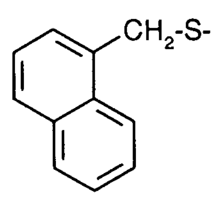 | $CH_3$ | $CH_3$ | $BF_4^-$ | Fp. 152° |
| 15 | 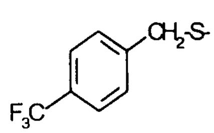 | $CH_3$ | $CH_3$ | $BF_4^-$ | Fp. 129°C |

| Bsp.-Nr. | R | $R^1$ | $R^2$ | X | physikalische Eigenschaften |
|---|---|---|---|---|---|
| 16 | 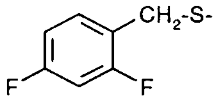 | $CH_3$ | $CH_3$ | $BF_4^-$ | Fp. 106-108°C |
| 17 | $-N(CH_3)_2$ | $-CH_2-(CH_2)_4-CH_2-$ | | $BF_4^-$ | $^1$H-NMR*): 3,25; 3,4 (d, 6H) |
| 18 | $-N(CH_3)_2$ | $C_2H_5$ | $n-C_4H_9$ | $I^-$ | Fp. 88-92°C (Zers.) |
| 19 | $-N(CH_3)_2$ | $-CH_2-CH=CH_2$ | $-CH_2-CH=CH_2$ | $I^-$ | $^1$H-NMR*): 3,3; 3,45 (d, 6H) |
| 20 | $-N(CH_3)_2$ | $-CH_2-C_6H_5$ | $-CH_2-C_6H_5$ | $I^-$ | Fp. 171-174°C (Zers.) |
| 21 | $-N(CH_3)_2$ | $C_2H_5$ | $-CH_2-C_6H_5$ | $I^-$ | $^1$H-NMR*): 3,3; 3,45 (d, 6H) |
| 22 | $-N(CH_3)_2$ | $n-C_4H_9$ | $-CH_2-C_6H_5$ | $I^-$ | $^1$H-NMR*): 3,3; 3,45 (d, 6H) |
| 23 | $-N(CH_3)_2$ | $n-C_4H_9$ | $-CH_2-C_6H_5$ | $BF_4^-$ | $^1$H-NMR*): 3,3; 3,45 (d, 6H) |
| 24 | $-N(CH_3)_2$ | $CH_3$ | $-CH_2-C_6H_5$ | $I^-$ | Fp. 118-121°C (Zers.) |
| 25 | $-N(CH_3)_2$ | $CH_3$ | $C_2H_5$ | $I^-$ | Fp. 82-85°C (Zers.) |
| 26 | $-N(CH_3)_2$ | $CH_3$ | $n-C_3H_7$ | $I^-$ | Fp. 103-105°C (Zers.) |
| 27 | $-N(CH_3)_2$ | $n-C_3H_7$ | $n-C_3H_7$ | $BF_4^-$ | Fp. 102-106°C |
| 28 | $-N(CH_3)_2$ | $-CH_2-C_6H_5$ | $-CH_2-CH_2-C_6H_5$ | $I^-$ | Fp. 119-121°C (Zers.) |
| 29 | $-N(CH_3)_2$ | $CH_3$ | 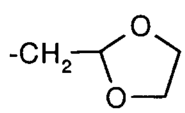 | $I^-$ | Fp. 91-94°C (Zers.) |

| Bsp.-Nr. | R | $R^1$ | $R^2$ | X | physikalische Eigenschaften |
|---|---|---|---|---|---|
| 30 | $-N(CH_3)_2$ | $CH_3$ | $n-C_6H_{13}$ | $I^-$ | Fp. 108-111°C (Zers.) |
| 31 | $-N(CH_3)_2$ | $CH_3$ | $-CH_2-CH_2-CN$ | $I^-$ | Fp. 126°C (Zers.) |
| 32 | $-N(CH_3)_2$ | $-CH_2-C_6H_5$ | $-(CH_2)_2-N(CH_3)_2$ | $BF_4^-$ | $^1$H-NMR*): 3,25; 3,4 (d, 6H) |
| 33 | $-N(CH_3)_2$ | $C_2H_5$ | $-(CH_2)_2-N(CH_3)_2$ | $BF_4^-$ | $^1$H-NMR*): 3,25; 3,4 (d, 6H) |
| 34 | $-N(CH_3)_2$ | $i-C_3H_7$ | $-CH_2-C_6H_5$ | $I^-$ | Fp. 112-116°C |
| 35 | $-SCH_3$ | $-CH_2-(CH_2)_3-CH_2-$ | | $I^-$ | $^1$H-NMR*): 2,65 (s, 3H) |
| 36 | $-N(CH_3)_2$ | $CH_3$ | $i-C_3H_7$ | $I^-$ | Fp. 99-103°C (Zers.) |
| 37 | $-N(CH_3)_2$ | $C_2H_5$ | $i-C_3H_7$ | $I^-$ | $^1$H-NMR*): 3,25; 3,4 (d, 6H) |
| 38 | $-N(CH_3)_2$ | $CH_3$ | $t-C_4H_9$ | $I^-$ | Fp. 96°C (Zers.) |
| 39 | $-N(CH_3)_2$ | $CH_3$ | $n-C_5H_{11}$ | $I^-$ | $^1$H-NMR*): 3,25; 3,4 (d, 6H) |
| 40 | $-N(CH_3)_2$ | $n-C_3H_7$ | 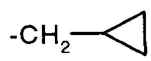 | $I^-$ | $^1$H-NMR*): 3,25; 3,4 (d, 6H) |
| 41 | $-N(CH_3)_2$ | $C_2H_5$ | $n-C_3H_7$ | $I^-$ | $^1$H-NMR*): 3,25; 3,4 (d, 6H) |
| 42 | $-N(CH_3)_2$ | $n-C_3H_7$ | $-CH_2-C_6H_5$ | $I^-$ | Fp. 103-108°C (Zers.) |
| 43 | $-N(CH_3)_2$ | $CH_3$ | 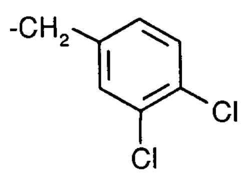 | $I^-$ | Fp. 103-108°C (Zers.) |

| Bsp.-Nr. | R | $R^1$ | $R^2$ | X | physikalische Eigenschaften |
|---|---|---|---|---|---|
| 44 | $-N(CH_3)_2$ | $CH_3$ | 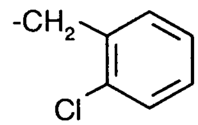 | $I^-$ | Fp. 116°C (Zers.) |
| 45 | $-N(CH_3)_2$ | $CH_3$ | $i-C_4H_9$ | $I^-$ | $^1H$-NMR*): 3,4 (s, 6H) |
| 46 | $-N(CH_3)_2$ | $CH_3$ | 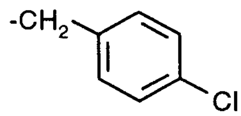 | $I^-$ | Fp. 112°C (Zers.) |
| 47 | $-N(CH_3)_2$ | $CH_3$ | $n-C_{12}H_{25}$ | $I^-$ | Fp. 103-109°C (Zers.) |
| 48 | $-N(CH_3)_2$ | $CH_3$ | 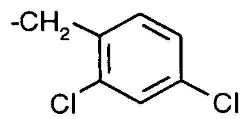 | $I^-$ | Fp. 127°C (Zers.) |
| 49 | $-N(CH_3)_2$ | $C_2H_5$ | 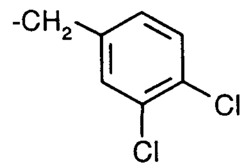 | $BF_4^-$ | $^1H$-NMR*): 3,25; 3,3 (d, 6H) |
| 50 | $-N(CH_3)_2$ | $C_2H_5$ | 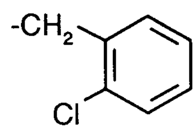 | $I^-$ | Fp. 97-101°C (Zers.) |
| 51 | $-N(CH_3)_2$ | $C_2H_5$ | 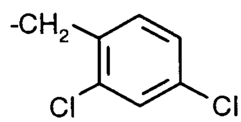 | $I^-$ | Fp. 133°C (Zers.) |
| 52 | $-N(CH_3)_2$ | $C_2H_5$ | 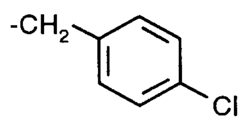 | $I^-$ | Fp. 96-101°C (Zers.) |
| 53 | $-N(CH_3)_2$ | $C_2H_5$ | 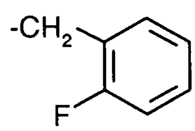 | $I^-$ | Fp. 85-88°C (Zers.) |
| 54 | $-N(CH_3)_2$ | $C_2H_5$ | 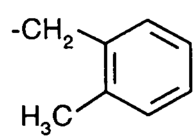 | $I^-$ | Fp. 104-109°C (Zers.) |

| Bsp.-Nr. | R | $R^1$ | $R^2$ | X | physikalische Eigenschaften |
|---|---|---|---|---|---|
| 55 | $-N(CH_3)_2$ | $C_2H_5$ | 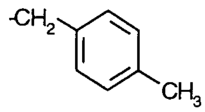 | $I^-$ | Fp. 106-111°C (Zers.) |
| 56 | $-N(CH_3)_2$ | $t\text{-}C_4H_9$ | $-CH_2\text{-}C_6H_5$ | $I^-$ | Fp. 112°C (Zers.) |
| 57 | $-N(CH_3)_2$ | $C_2H_5$ | $-CH_2\text{-}CH{=}CH_2$ | I | Fp. 167°C (Zers.) |
| 58 | $-N(CH_3)_2$ | $CH_3$ | 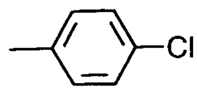 | I | Fp. 138°C (Zers.) |
| 59 | $-N(CH_3)_2$ | $CH_3$ | 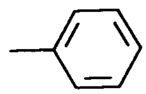 | I | Fp. 144-147°C (Zers.) |
| 60 | $-N(CH_3)_2$ | $CH_3$ | 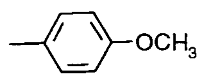 | I | Fp. 173°C (Zers.) |
| 61 | $-N(CH_3)_2$ | $-CH_2\text{-}CH{=}CH_2$ | 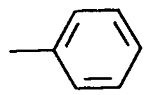 | I | $^1$H-NMR δ= 3.25; 3.4 (d, 6H) |
| 62 | $-N(CH_3)_2$ | $C_2H_5$ | 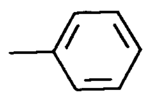 | I | Fp. 131-136°C (Zers.) |
| 63 | $-N(CH_3)_2$ | $-CH_2CH_2CN$ | 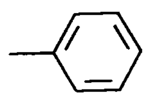 | I | Fp. 178°C (Zers.) |
| 64 | $-N(CH_3)_2$ | 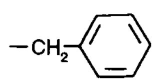 | 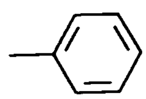 | I | Fp. 66°C (Zers.) |

| Bsp.-Nr. | R | $R^1$ | $R^2$ | X | physikalische Eigenschaften |
|---|---|---|---|---|---|
| 65 | $-N(CH_3)_2$ | $-CH(CH_3)_2$ | 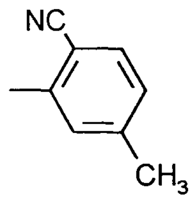 | I | $^1$H-NMR δ=3.25;3.35 (d, 6H) |
| 66 | $-N(CH_3)_2$ | $n-C_4H_9$ | 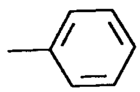 | I | $^1$H-NMR δ=3.3;3.45 (d, 6H) |
| 67 | $-N(CH_3)_2$ | $C_2H_5$ | 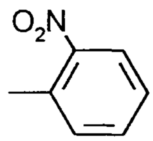 | I | Fp. 112-116°C (Zers.) |
| 68 | $-N(CH_3)_2$ | $CH_3$ | 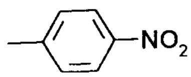 | I | Fp. 174-177°C (Zers.) |
| 69 | $-N(CH_3)_2$ | $CH_3$ | 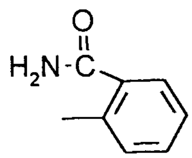 | I | Fp. 141-145°C (Zers.) |
| 70 | $-N(CH_3)_2$ | $CH_3$ | 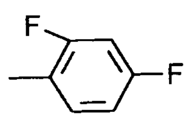 | I | Fp. 192-195°C (Zers.) |
| 71 | $-N(CH_3)_2$ | $CH_3$ | 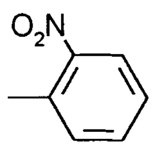 | I | Fp. 119-125°C (Zers.) |
| 72 | $-N(CH_3)_2$ | $CH_3$ | 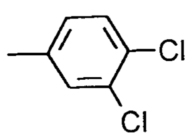 | I | Fp. 128°C (Zers.) |
| 73 | $-N(CH_3)_2$ | $CH_3$ | 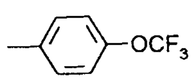 | I | $^1$H-NMR δ= 3.3; 3.5 (d, 6H) |
| 74 | $-N(CH_3)_2$ | $CH_3$ | 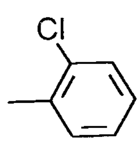 | I | Fp. 189°C (Zers.) |

| Bsp.-Nr. | R | $R^1$ | $R^2$ | X | physikalische Eigenschaften |
|---|---|---|---|---|---|
| 75 | $-N(CH_3)_2$ | $CH_3$ | 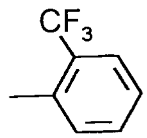 | I | Fp. 134-138°C (Zers.) |
| 76 | $-N(CH_3)_2$ | $C_2H_5$ | 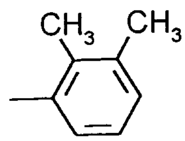 | I | Fp. 189°C (Zers.) |
| 77 | $-N(CH_3)_2$ | $CH_3$ | $CH_3$ | I | Fp. 231°C (Zers.) |
| 78 | $-N(CH_3)_2$ | $CH_2CH_2NEt_2$ | 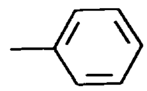 | I | $^1$H-NMR δ= 3.3; 3.45 (d, 6H) |
| 79 | $C_2H_5S-$ | $CH_3$ | $CH_3$ | I | Fp. 53-59°C |
| 80 | $nC_3H_7S-$ | $CH_3$ | $CH_3$ | I | Fp. 123-127°C |
| 81 | $iC_3H_7S-$ | $CH_3$ | $CH_3$ | I | Fp. 141-144°C |
| 82 | $nC_4H_9S-$ | $CH_3$ | $CH_3$ | I | Fp. 99-101°C |
| 83 | $-N(CH_3)_2$ | 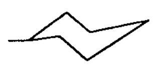 | 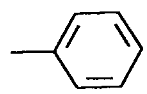 | I | Fp. 179°C (Zers.) |
| 84 | $-N(CH_3)_2$ | $CH_2CH_2NMe_2$ | 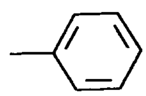 | I | $^1$H-NMR δ= 3.35; 3.45 (d, 6H) |
| 85 | $-N(CH_3)_2$ | $CH_3$ | 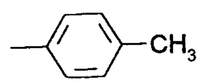 | I | Fp. 135°C (Zers.) |
| 86 | $-N(CH_3)_2$ | $nC_3H_7$ | 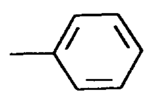 | I | $^1$H-NMR δ= 3.3; 3.4 (d, 6H) |
| 87 | $-N(CH_3)_2$ | $iC_3H_7$ | 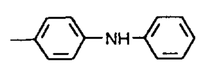 | I | Fp. 86°C (Zers.) |
| 88 | $-N(CH_3)_2$ | $C_2H_5$ | 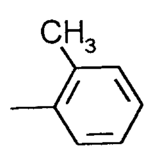 | I | Fp. 93°C (Zers.) |

| Bsp.-Nr. | R | $R^1$ | $R^2$ | X | physikalische Eigenschaften |
|---|---|---|---|---|---|
| 89 | $(CH_3)_2CHCH_2S-$ | $CH_3$ | $CH_3$ | I | Fp. 119-123°C (Zers.) |
| 90 | $(CH_3)_3C-S-$ | $CH_3$ | $CH_3$ | I | Fp. 167°C (Zers.) |
| 91 | 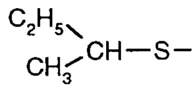 | $CH_3$ | $CH_3$ | I | Fp. 91-96°C (Zers.) |
| 92 | $nC_5H_{11}S-$ | $CH_3$ | $CH_3$ | I | Fp. 128-132°C |
| 93 | $(CH_3)_2CHCH_2CH_2S-$ | $CH_3$ | $CH_3$ | I | Fp. 128-132°C |
| 94 | $nC_4F_9S-$ | $CH_3$ | $CH_3$ | I | Fp. 198-201°C |
| 95 | $-N(CH_3)_2$ | 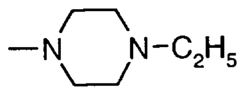 | | I | Fp. 177°C (Zers.) |
| 96 | $-N(CH_3)_2$ | 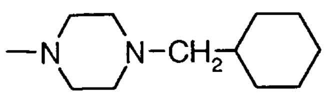 | | I | Fp. 229°C (Zers.) |

*) Die $^1$H-NMR-Spektren wurden in Hexadeutero-Dimethylsulfoxid (DMSO-$d_6$) mit Tetramethylsilan (TMS) als innerem Standard aufgenommen. Angegeben ist die chemische Verschiebung als d-Wert in ppm.

## Patentansprüche

1. Arzneimittel enthaltend 1,2,4-Dithiazolium-Salze der allgemeinen Formel (I),

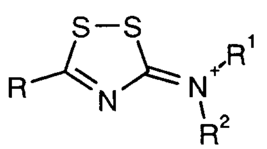 $X^-$ (I)

in welcher

| | |
|---|---|
| R | für einen Rest der Formel $-S-R^3$ oder $-NR^4R^5$ steht, |
| $R^1$ | für Alkyl oder Aryl steht und |
| $R^2$ | für Alkyl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann und |
| X | für das Anion einer anorganischen Säure steht, wobei |
| $R^3$ | für gegebenenfalls substituiertes Alkyl steht, |
| $R^4$ | für jeweils gegebenenfalls substituiertes Alkyl, Alkenyl, Alkinyl oder Aryl steht und |
| $R^5$ | für jeweils gegebenenfalls substituiertes Alkyl oder Alkenyl steht oder |
| $R^4$ und $R^5$ | gemeinsam mit dem Stickstoffatom, an welches sie gebunden sind, für einen gegebenenfalls substituierten Heterocyclus stehen, der gegebenenfalls weitere Heteroatome |

enthalten kann.

**2.** Arzneimittel enthaltend Verbindungen der Formel (I) gemäß Anspruch 1, bei welchen

R für einen Rest der Formel $-S-R^3$ oder $-NR^4R^5$ steht,

$R^1$ für geradkettiges oder verzweigtes Alkyl mit 1 bis 8 Kohlenstoffatomen oder für gegebenenfalls einfach oder mehrfach, gleich oder verschieden substituiertes Aryl mit 6 bis 10 Kohlenstoffatomen steht, wobei als Substituenten jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl und/oder geradkettiges oder verzweigtes Alkoxy und/oder geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl und

$R^2$ für geradkettiges oder verzweigtes Alkyl mit 1 bis 8 Kohlenstoffatomen steht oder

$R^1$ und $R^2$ gemeinsam mit dem Stickstoffatom, an welches sie gebunden sind für einen gegebenenfalls einfach oder mehrfach, gleich oder verschieden substituierten, gesättigten fünf- bis siebengliedrigen Heterocyclus stehen, der gegebenenfalls weitere Heteroatome - insbesondere Stickstoff, Sauerstoff und/oder Schwefel - enthalten kann, wobei als Substituenten infrage kommen: Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen,

X für das Anion einer anorganischen Säure steht, wobei

$R^3$ für geradkettiges oder verzweigtes Alkyl mit 1 bis 8 Kohlenstoffatomen oder für jeweils gegebenenfalls im Arylteil einfach oder mehrfach, gleich oder verschieden substituiertes Arylalkyl mit 6 bis 10 Kohlenstoffatomen im Arylteil und 1 bis 4 Kohlenstoffatomen im geradkettigen oder verzweigten Alkylteil oder Heteroarylalkyl mit 2 bis 9 Kohlenstoffatomen und 1 bis 3 gleichen oder verschiedenen Heteroatomen - insbesondere Stickstoff, Sauerstoff und/oder Schwefel - im Heteroarylteil und 1 bis 4 Kohlenstoffatomen im geradkettigen oder verzweigten Alkylteil steht, wobei als Substituenten jeweils infrage kommen:

Halogen, Cyano, Nitro, Carbamoyl, jeweils geradkettiges oder verzweigtes Alkyl, Alkoxy oder Alkoxycarbonyl mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen,

$R^4$ für jeweils geradkettiges oder verzweigtes Alkyl, Alkenyl oder Alkinyl mit jeweils bis zu 8 Kohlenstoffatomen, für geradkettiges oder verzweigtes Halogenalkyl mit 1 bis 8 Kohlenstoffatomen und 1 bis 17 gleichen oder verschiedenen Halogenatomen, für jeweils geradkettiges oder verzweigtes Cyanalkyl, Dioxolanylalkyl, Alkoxyalkyl oder Dialkylaminoalkyl mit jeweils 1 bis 8 Kohlenstoffatomen in den einzelnen Alkylteilen, für Cycloalkylalkyl mit 3 bis 8 Kohlenstoffatomen im Cycloalkylteil und 1 bis 8 Kohlenstoffatomen im geradkettigen oder verzweigten Alkylteil oder für jeweils gegebenenfalls im Arylteil einfach oder mehrfach, gleich oder verschieden substituiertes Aryl mit 6 bis 10 Kohlenstoffatomen, Arylalkyl mit 6 bis 10 Kohlenstoffatomen im Arylteil und 1 bis 4 Kohlenstoffatomen im geradkettigen oder verzweigten Alkylteil oder Heteroarylalkyl mit 2 bis 9 Kohlenstoffatomen und 1 bis 3 gleichen oder verschiedenen Heteroatomen - insbesondere Stickstoff, Sauerstoff und/oder Schwefel - im Heteroarylteil und 1 bis 4 Kohlenstoffatomen im geradkettigen oder verzweigten Alkylteil steht, wobei als Substituenten jeweils infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen und

$R^5$ für jeweils geradkettiges oder verzweigtes Alkyl oder Alkenyl mit jeweils bis zu 8

Kohlenstoffatomen steht oder für gegebenenfalls im Arylteil einfach oder mehrfach, gleich oder verschieden substituiertes Arylalkyl mit 6 bis 10 Kohlenstoffatomen im Arylteil und 1 bis 4 Kohlenstoffatomen im geradkettigen oder verzweigten Alkylteil steht, wobei als Substituenten infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder

$R^4$ und $R^5$ gemeinsam mit dem Stickstoffatom, an welches sie gebunden sind für einen gegebenenfalls einfach oder mehrfach, gleich oder verschieden substituierten, gesättigten fünf- bis siebengliedrigen Heterocyclus stehen, der gegebenenfalls weitere Heteroatome - insbesondere Stickstoff, Sauerstoff und/oder Schwefel - enthalten kann, wobei als Substituenten infrage kommen:

Halogen, jeweils geradkettiges oder verzweigtes Alkyl oder Alkoxy mit jeweils 1 bis 4 Kohlenstoffatomen oder jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen.

**3.** Arzneimittel enthaltend Verbindungen der Formel (I), bei welchen

$R$ für einen Rest der Formel $-S-R^3$ oder $-NR^4R^5$ steht,

$R^1$ für geradkettiges oder verzweigtes Alkyl mit 1 bis 6 Kohlenstoffatomen oder für gegebenenfalls einfach bis dreifach, gleich oder verschieden substituiertes Phenyl steht, wobei als Substituenten jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen,

$R^2$ für geradkettiges oder verzweigtes Alkyl mit 1 bis 6 Kohlenstoffatomen steht oder

$R^1$ und $R^2$ gemeinsam mit dem Stickstoffatom, an welches sie gebunden sind für einen gegebenenfalls einfach bis vierfach, gleich oder verschieden substituierten, gesättigten fünf- bis siebengliedrigen Heterocyclus stehen, der gegebenenfalls ein weiteres Heteroatom - insbesondere Stickstoff, Sauerstoff oder Schwefel - enthalten kann, wobei als Substituenten infrage kommen:

Halogen oder jeweils geradkettiges oder verzweigtes Alkyl oder Alkoxy mit jeweils 1 bis 4 Kohlenstoffatomen,

$X$ für ein Halogenidanion, ein Phosphatanion, ein Hexafluorophosphatanion, ein Sulfatanion, ein Hydrogensulfatanion, ein Nitratanion, ein Carbonatanion, ein Perchloratanion oder ein Tetrafluoroboratanion steht, wobei

$R^3$ für geradkettiges oder verzweigtes Alkyl mit 1 bis 4 Kohlenstoffatomen oder für jeweils gegebenenfalls im Arylteil einfach bis dreifach, gleich oder verschieden substituiertes Arylalkyl mit 6 oder 10 Kohlenstoffatomen im Arylteil und 1 oder 2 Kohlenstoffatomen im Alkylteil oder Heteroarylalkyl mit 2 bis 5 Kohlenstoffatomen und 1 bis 3 gleichen oder verschiedenen Heteroatomen - insbesondere Stickstoff, Sauerstoff und/oder Schwefel - im Heteroarylteil und 1 oder 2 Kohlenstoffatomen im Alkylteil steht, wobei als Substituenten jeweils infrage kommen:

Fluor, Chlor, Brom, Iod, Cyano, Nitro, Carbamoyl, jeweils geradkettiges oder verzweigtes Alkyl, Alkoxy oder Alkoxycarbonyl mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen,

$R^4$ für jeweils geradkettiges oder verzweigtes Alkyl, Alkenyl oder Alkinyl mit jeweils bis zu 6 Kohlenstoffatomen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Cyanalkyl, Dioxolanylalkyl, Alkoxyalkyl oder Dialkylaminoalkyl mit jeweils 1 bis 4 Kohlenstoffatomen in den einzelnen Alkylteilen, für Cycloalkylalkyl mit 3 bis 7 Kohlenstoffatomen im Cycloalkylteil und 1 bis 4 Kohlenstoffatomen im geradkettigen oder verzweigten Alkylteil oder für jeweils gegebenenfalls im Arylteil einfach bis dreifach, gleich oder verschieden substituiertes Aryl mit 6 oder 10 Kohlenstoffatomen, Arylalkyl mit 6 oder 10 Kohlenstoffatomen im Arylteil und 1 oder 2 Kohlenstoffatomen im Alkylteil oder Heteroarylalkyl mit 2 bis 5 Kohlenstoffatomen und 1 bis 3 gleichen oder verschiedenen Heteroatomen - insbesondere Stickstoff, Sauerstoff und/oder Schwefel - im Heteroarylteil und 1 oder 2 Kohlenstoffatomen im Alkylteil steht, wobei als Substituenten jeweils infrage kommen:
Fluor, Chlor, Brom, Iod, jeweils geradkettiges oder verzweigtes Alkyl oder Alkoxy mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen und

$R^5$ für jeweils geradkettiges oder verzweigtes Alkyl oder Alkenyl mit jeweils bis zu 6 Kohlenstoffatomen steht oder für gegebenenfalls im Arylteil einfach bis dreifach, gleich oder verschieden substituiertes Arylalkyl mit 6 oder 10 Kohlenstoffatomen im Arylteil und 1 oder 2 Kohlenstoffatomen im Alkylteil steht, wobei als Substituenten infrage kommen:
Fluor, Chlor, Brom, Iod, jeweils geradkettiges oder verzweigtes Alkyl oder Alkoxy mit jeweils 1 bis 3 Kohlenstoffatomen oder jeweils geradkettiges oder verzweigtes Halogenalkyl oder Halogenalkoxy mit jeweils 1 bis 3 Kohlenstoffatomen und 1 bis 7 gleichen oder verschiedenen Halogenatomen oder

$R^4$ und $R^5$ gemeinsam mit dem Stickstoffatom, an welches sie gebunden sind für einen gegebenenfalls einfach bis vierfach, gleich oder verschieden substituierten, gesättigten fünf- bis siebengliedrigen Heterocyclus stehen, der gegebenenfalls ein weiteres Heteroatom - insbesondere Stickstoff, Sauerstoff oder Schwefel - enthalten kann, wobei als Substituenten infrage kommen:
Halogen oder jeweils geradkettiges oder verzweigtes Alkyl oder Alkoxy mit jeweils 1 bis 4 Kohlenstoffatomen.

**4.** Arzneimittel enthaltend Verbindungen der Formel (I) gemäß Anspruch 1, bei welchen

R für einen Rest der Formel $-S-R^3$ oder $-NR^4R^5$ steht,

$R^1$ für geradkettiges oder verzweigtes Alkyl mit 1 bis 4 Kohlenstoffatomen oder für gegebenenfalls einfach oder zweifach, gleich oder verschieden substituiertes Phenyl steht, wobei als Substituenten jeweils infrage kommen:
Fluor, Chlor, Brom, Cyano, Nitro, Methyl, Ethyl, n-oder i-Propyl, n-, i-, s-oder t-Butyl, Methoxy, Ethoxy, n-oder i-Propoxy, n-, i-, s-oder t-Butoxy, Trifluormethyl, Difluormethyl, Trifluormethoxy oder Difluormethoxy,

$R^2$ für geradkettiges oder verzweigtes Alkyl mit 1 bis 4 Kohlenstoffatomen steht oder

$R^1$ und $R^2$ gemeinsam mit dem Stickstoffatom, an welches sie gebunden sind für einen gegebenenfalls einfach oder zweifach substituierten Pyrrolidinyl-, Piperidinyl-, Perhydroazepinyl-, Piperazinyl-oder Morpholinylrest stehen, wobei als Substituenten jeweils infrage kommen:
Chlor, Methyl, Ethyl, Methoxy oder Ethoxy,

X für ein Fluorid-, Chlorid-, Bromid-, Iodid- oder Tetrafluoroboratanion steht, wobei

$R^3$ für Methyl, Ethyl oder für jeweils gegebenenfalls im Arylteil einfach oder zweifach, gleich oder verschieden substituiertes Phenyl, Naphthyl, Benzyl oder Pyridylmethyl steht, wobei als Substituenten jeweils infrage kommen:
Fluor, Chlor, Brom, Cyano, Nitro, Methyl, Ethyl, n-oder i-Propyl, Methoxy, Ethoxy, n-oder i-Propoxy, Methoxycarbonyl, Ethoxycarbonyl, Carbamoyl, Trifluormethyl, Difluormethyl, Trifluormethoxy oder Difluormethoxy,

$R^4$ für Methyl, Ethyl, n- oder i-Propyl, n-, i-, s- oder t-Butyl, Allyl, Propargyl, Cyanethyl, Methoxyethyl, Ethoxyethyl, Dimethylaminoethyl, Diethylaminoethyl, Dioxolanylmethyl, Cyclopropylmethyl, Cyclohexylmethyl oder für jeweils gegebenenfalls im Arylteil einfach oder zweifach, gleich oder verschieden substituiertes Phenyl, Naphthyl, Benzyl, Phenylethyl oder Pyridylmethyl steht, wobei als Substituenten jeweils infrage kommen:
Fluor, Chlor, Brom, Methyl, Ethyl, n-oder i-Propyl, Methoxy, Ethoxy, n-oder i-Propoxy, Trifluormethyl, Difluormethyl, Trifluormethoxy oder Difluormethoxy und

$R^5$ für Methyl, Ethyl, n- oder i-Propyl, n-, i-, s- oder t-Butyl, Allyl oder für gegebenenfalls im Arylteil einfach oder zweifach, gleich oder verschieden substituiertes Benzyl steht,

wobei als Substituenten infrage kommen:
Fluor, Chlor, Brom, Methyl, Ethyl, n-oder i-Propyl, Methoxy, Ethoxy, n-oder i-Propoxy, Trifluormethyl, Difluormethyl, Trifluormethoxy oder Difluormethoxy oder

$R^4$ und $R^5$ gemeinsam mit dem Stickstoffatom, an welches sie gebunden sind für einen gegebenenfalls einfach oder zweifach substituierten Pyrrolidinyl-, Piperidinyl-, Perhydroazepinyl-, Piperazinyl-oder Morpholinylrest stehen, wobei als Substituenten jeweils infrage kommen:
Chlor, Methyl, Ethyl, Methoxy oder Ethoxy.

**5.** Arzneimittel gemäß Ansprüchen 1 bis 4 zur Inhibition von TNF.

**6.** Verfahren zur Herstellung von Verbindungen gemäß den Ansprüchen 1 bis 4, dadurch gekennzeichnet, daß man 1,2,4-Dithiazolin-5-thione der Formel (II),

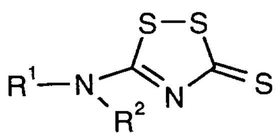 (II)

in welcher
$R^1$ und $R^2$ die in den Ansprüchen 1 bis 4 angegebene Bedeutung haben,
mit Alkyliodiden der Formel (III),

$R^3$- I (III)

in welcher
$R^3$ die in den Ansprüchen 1 bis 4 angegebene Bedeutung hat,
gegebenenfalls in Gegenwart eines Verdünnungsmittels umsetzt und gegebenenfalls anschließend die so erhältlichen 1,2,4-Dithiazolium-Salze der Formel (Ia),

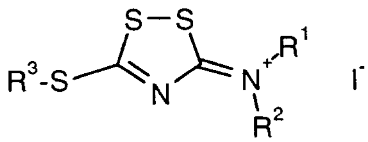 (Ia)

in welcher
$R^1$, $R^2$ und $R^3$ die in den Ansprüchen 1 bis 4angegebene Bedeutung haben,
in einer anschließenden zweiten Stufe mit Aminen der Formel (IV),

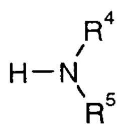 (IV)

in welcher
$R^4$ und $R^5$ die in den Ansprüchen 1 bis 4 angegebene Bedeutung haben,
gegebenenfalls in Gegenwart eines Verdünnungsmittels umsetzt und gegebenenfalls anschließend durch Umsetzung mit einer anorganischen Säure oder einem entsprechenden Salz das Anion austauscht.

**7.** 1,2,4-Dithiazolium-Salze der Formel (Ib),

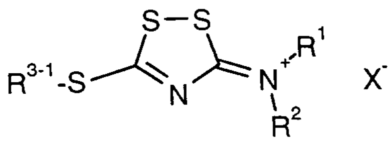

(Ib)

in welcher

| | |
|---|---|
| $R^1$ | für Alkyl oder Aryl steht und |
| $R^2$ | für Alkyl steht oder |
| $R^1$ und $R^2$ | gemeinsam mit dem Stickstoffatom, an welches sie gebunden sind, für einen gegebenenfalls substituierten Heterocyclus stehen, der gegebenenfalls weitere Heteroatome enthalten kann und |
| X | für das Anion einer anorganischen Säure steht, wobei |
| $R^{3-1}$ | für jeweils gegebenenfalls substituiertes Arylalkyl oder Heterocyclylalkyl steht. |

**8.** Arzneimittel enthaltend 1,2,4-Dithiazolium-Salze gemäß Anspruch 7.

Europäisches Patentamt

# EUROPÄISCHER RECHERCHENBERICHT

Nummer der Anmeldung
EP 94 10 8616

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | DE-A-22 22 201 (ORTHO PHARMACEUTICAL CORP.)<br>* das ganze Dokument *<br>--- | 1-5 | A61K31/41<br>C07D285/00<br>C07D417/12 |
| X | DE-A-22 19 992 (ORTHO PHARMACEUTICAL CORP.)<br>* das ganze Dokument *<br>--- | 1-5 | |
| D,X | JOURNAL OF MEDICINAL CHEMISTRY,<br>Bd.15, Nr.3, März 1972, WASHINGTON US<br>Seiten 315 - 320<br>J.E. OLIVER ET AL. 'Insect chemosterilants. 11. Substituted 3,5-diamino-1,2,4-dithiazolium salts and related compounds'<br>* das ganze Dokument, insbesondere Seite 315 *<br>--- | 6 | |
| D,P,X | WO-A-94 04517 (BAYER AKTIENGESELLSCHAFT)<br>* Ansprüche 5,11,12 *<br>--- | 6 | RECHERCHIERTE SACHGEBIETE (Int.Cl.5) |
| A | WO-A-91 10649 (FARMITALIA CARLO ERBA S.R.L.)<br>* das ganze Dokument, insbesonder Seite 11, Zeilen 21-31 *<br>----- | 1 | A61K<br>C07D |

Der vorliegende Recherchenbericht wurde für alle Patentansprüche erstellt

| Recherchenort | Abschlußdatum der Recherche | Prüfer |
|---|---|---|
| DEN HAAG | 15. September 1994 | Allard, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)